# EUROPÄISCHE PATENTANMELDUNG

(11) **EP 1 961 808 A2**
(43) Veröffentlichungstag der Anmeldung: **27.08.2008**
(21) Anmeldenummer: 08001777.5
(22) Anmeldetag: 22.05.2002
(51) Int. Cl.: C12N 5/00, C12N 5/06, D01F 9/08, D01F 1/10

(54) **Flächenelement und/oder dreidimensionales Raumelement aus Zellen**

(30) Priorität: 29.05.2001 DE 10126137
(62) Teilanmeldung aus: 02011295.9
(71) Anmelder: Bayer Innovation GmbH, 40225 Düsseldorf (DE)
(72) Erfinder: Thierauf, Axel, Dr., 84066 Mallersdorf-Pfaffenberg (DE); Haisch, Andreas, 12203 Berlin (DE)

(57) **Zusammenfassung**

Flächenelement und/oder dreidimensionales Raumelement aus Zellen, hergestellt mittels Zellen und einer Fasermatrix aus biologisch degradierbaren und/oder biologisch resorbierbaren Fasern, wobei die Fasermatrix als Zellstützsubstanz und/oder Leitstruktur für die von den Zellen gebildete extrazelluläre Matrix dient bzw. den Zellen die Möglichkeit gibt, eine räumliche Anordnung zu finden, die es den Zellen erlaubt, sich zu vermehren und/oder ihre genetisch determinierte Differenzierung zu erreichen.

## Beschreibung

Die Erfindung betrifft Flächenelemente und/oder dreidimensionales Raumelemente aus Zellen, hergestellt mittels Zellen und einer Fasermatrix aus biologisch degradierbaren und/oder biologisch resorbierbaren Fasern, wobei die Fasermatrix als Zellstützsubstanz und/oder Leitstruktur für die von den Zellen gebildete extrazelluläre Matrix dient bzw. den Zellen die Möglichkeit gibt, eine räumliche Anordnung zu finden, die es den Zellen erlaubt, sich zu vermehren und/oder ihre genetisch determinierte Differenzierung zu erreichen.

Als Zellen können beispielsweise undifferenzierte pluripotente Stammzellen oder gentechnisch veränderter oder nativer differenzierter Zellen verschiedener Differenzierungsarten und -grade verwendet werden, wobei die Zellen in eine Matrix/Fasem aus vorzugsweise Polykieselsäure mit unterschiedlicher räumlicher Anordnung ein- oder auf solche Fasern aufgebracht werden.

Vorzugsweise ist vorgesehen, dass bei der Herstellung eines Flächenelements aus Zellen, die Fasermatrix als Flächenelement, insbesondere in Form eines Vlieses oder Gewebes vorliegt, das aus biologisch degradierbaren und/oder biologisch resorbierbaren Fasern besteht, die man dadurch erhält, dass man aus einer Spinmasse Fäden zieht und diese gegebenenfalls trocknet, wobei die Spinnmasse eine oder mehrere partiell oder vollständig hydrolytisch kondensierte Verbindungen des Siliziums enthält, die sich durch hydrolytische Kondensation ableiten von Monomeren der allgemeinen Formel SiX₄, in der die Reste X gleich oder verschieden sind und Hydroxy, Wasserstoff, Halogen, Amino, Alkoxy, Alkyloxy, Alkylcarbonyl oder Alkoxycarbonyl bedeuten und sich von Alkyl-Resten ableiten, die gegebenenfalls substituierte geradkettige, verzweigte oder cyclische Reste mit 1 bis 20 Kohlenstoff-Atomen, bevorzugt mit 1 bis 10 Kohlenstoff-Atomen, darstellen und durch Sauerstoff oder Schwefel-Atome oder durch Amino-Gruppen unterbrochen sein können.

Eine derartige Faser wird hinsichtlich ihrer Eigenschaften und ihrer Herstellung in DE 196 09 551 C1 ausführlich beschrieben.

Solche Fasern sind biologisch degradierbar und biologisch resorbierbar und lösen sich in schwach basischen, körperähnlichen Flüssigkeiten mit Abbauraten von 10 nm bis 100 nm Faserradius pro Tag auf, wobei die Abbaurate mit der Zahl der Silanol-Gruppen der Faser korreliert ist.

Zur Herstellung von Geweben außerhalb des menschlichen Körpers kommen vielfältige Verfahren zur Anwendung, die unter dem weitläufigen Begriff "tissue engineering" zusammengefasst werden. Hierzu werden je nach Gewebeart Zellen aus ihrem bestehenden Gewebeverbund isoliert und zur Vermehrung gebracht. Hiernach werden die Zellen entweder auf flächige Materialien unterschiedlicher Konsistenz aufgebracht oder in poröse bzw. gelartige Materialien eingebracht, hierdurch die Gewebereifung induziert und ggf. durch Differenzierungsfaktoren stimuliert. Die Gewebereifung kann außerhalb oder innerhalb des Körpers erfolgen. Als Trägermaterialien stehen bioresorbierbare und nicht-bioresorbierbare, organische und anorganische Stoffe zur Verfügung.

Bioresorbierbare Materialien haben zum Vorteil, daß idealerweise ein rückstandsfreier Abbau der Trägerstrukur erfolgt. Nachteile derzeitig verwendeter bioresorbierbarer organischer Materialien (z.B. Polylactide, Polyglycolide, Milchsäurederivate) sind die Änderung des Gewebemilieus durch Abbauprozesse und Abbauprodukte mit negativer Beeinflussung der Gewebereifung sowie die Infektionsgefahr durch Verwendung bovinen und humanen Materials (HIV, BSE etc.). Weiterhin induzieren die derzeit verwendeten bioresorbierbaren organischen Materialien im menschlichen Körper eine Fremdkörperreaktion mit Abkapselung und partieller Persistenz von Materialbestandteilen und begünstigen eine überschießende narbige Wundheilung mit der Folge einer negativen Beeinflussung der Gewebereifung.

Nicht-bioresorbierbare anorganische Materialien (z.B. Polyamide, Nylon, Teflon) bleiben dauerhaft vorhanden, da sie weder außerhalb noch innerhalb des Körpers abgebaut werden. Dies hat zum Vorteil, daß keine die Gewebereifung negativ beeinflussenden Abbauprodukte entstehen. Nachteilig für die Versorgung und die Reifung der Gewebe zeigen sich jedoch innerhalb des Körpers Fremdkörperreaktionen und narbige Kapselbildungen mit Einschränkung der Gewebeversorgung. Ein weiterer Nachteil der nicht-bioresorbierbaren Materialien besteht in der deutlich erhöhten Infektionsanfälligkeit und der nur unzulänglichen Erreichbarkeit für anti-infektiöse Medikamente im Falle einer ungewollten Besiedlung mit Erregern (= Infektion). Ein weiterer Nachteil für die Eigenschaften des neu gebildeten oder neu zu bildenden Gewebes kann die Tatsache sein, daß die Materialeigenschaften der Trägerstruktur in die Gesamteigenschaften des neu gebildeten Gewebes eingehen.

Die US-Patente 5,736,372 und 5,855,610 beschreiben die Verwendung vielfältiger Fasermaterialien zur Herstellung von festen Geweben (dreidimensional). Ein Material, wie es erfindungsgemäß Verwendung findet, ist dort jedoch nicht beschrieben.
Bekannt ist aus der DE-C 196 09 551 ferner ein Faser-Material auf Si-Basis, das durch partielle oder vollständige hydrolytische Kondensation von einer oder mehreren Si-Verbindungen als Spinnmasse erhalten und anschließend zu Fasern verarbeitet wird.

Aufgabe der vorliegenden Erfindung war es deshalb Flächenelemente oder dreidimensionales Raumelemente aus Zellen herzustellen, um sie anschließend ggf. in den Körper eines Menschen zu bringen. Diese Aufgabe haben die Erfinder dadurch gelöst, daß sie das aus der DE-C 196 09 551 bekannte Faser-Material als Matrix für die flächige und/oder dreidimensionale Anwendung heranziehen. Das erfindungsgemäß verwendete Biomaterial wird ohne Änderung des Gewebemilieus und ohne Fremdkörperreaktion vollständig im Organismus abgebaut und vermeidet somit die Nachteile der in den US-Patentschriften 5,736,372 und 5,855,610 beschriebenen organischen bioresorbierbaren und nicht-bioresorbierbaren Polymermatrices.

Eine genauere Beschreibung der verwendeten Fasertypen wird nachfolgend an Hand physikalischer Größen gegeben. Die Eigenschaftsprofile der Fasern sind (bei einer Fehlerstreuung von 25 bis 35%)
1) Faserparameter:
   - mittlere Zugfestigkeit: bis zu 300 MPa (Einzelwerte bis 465 MPa)
   - E-Modul: bis zu 17 GPa
   - Bruchdehnung: um 2 %
   - OH-Gehalt: ca. 5 % nach TGA
   - spezifische Oberfläche 0,117 m²/g
   - Porosität: mit BET nicht bestimmbar
   - Querschnittsflächen: 100 - 400 µm²
   Auf Grund der Fehlerstreuung kann der E-Modul beispielsweise Werte von 11 bis zu 23 GPa erreichen (17 ± 35%).
2) Cytotoxikologischer Test nach ISO 10993/EN 30993
   - direkter Kontakt-Test mit L929-Zellen, Fibroblasten-Zellinie der Maus
   - Extrakt-Test mit MTT
   - Extrakt-Test mit BrdU ergab:

   - Enzymaktivität: 67 % gegen 92 % für negatives Referenzmaterial
   - Zellproliferation: 99 % gegen 100 % für negatives Referenzmaterial
   Je nach Zellart müssen die Zellen entweder zuvor durch enzymatischen Verdau oder durch mechanische Trennung aus ihrem Matrixverbund gelöst werden oder durch Auf- oder Einbringen auf/in ein Nährmedium unter physiologischen Bedingungen zum Wachstum angeregt werden. Die o.g. Fasermatrix fungiert hierbei als Leitstruktur für das Zellwachstum oder als Leitstruktur für die Akkumulation extrazellulärer Matrix- und Gewebebestandteile.

Erfindungsgemäß kann das Faser-Material in verschiedenen Anordnungen zur Anwendung kommen. Welche Anordnung zu wählen ist, weiß der Fachmann an Hand des herzustellenden (Zell-)Gewebes zu bestimmen. Die in Betracht kommenden Anordnungen sind die folgenden:
1) als Flächenelement, d.h. als dichtes Fasernetz, das zwar eine über die Dimension der aufgebrachten Zellen hinausgehende, aber doch nur eine begrenzte Penetration ermöglicht (d.h. die durchschnittliche Größe der Löcher/Faser- bzw. Netzzwischenräume ist keinesfalls größer, bevorzugt sogar kleiner als die durchschnittliche Größe der zu kultivierenden Zellen; somit können die Zellen zwar "in die Fasern einwachsen", dies aber nur derart, daß sie auf der Unterlage der Fasern gut haften), mit der im wesentlichen alleinigen, zumindest aber vornehmlichen Möglichkeit der zweidimensionalen Zellverteilung und einem flächigen Zell,- Gewebe- und Organwachstum;
2) als dreidimensionales Raumelement, d.h. als poröses, von den Zellen penetrierbares Fasernetz (d.h. die durchschnittliche Größe der Löcher/Faser- bzw. Netzzwischenräume ist keinesfalls kleiner, bevorzugt sogar größer als die durchschnittliche Größe der zu kultivierenden Zellen) mit der Möglichkeit der dreidimensionalen Zellverteilung und einem räumlichen Zell,-Gewebe- und Organwachstum;
3) als Kombination von 1) und 2) im Sinne eines "composite graft" oder Orkanes durch Kombination von Zellen, Geweben bzw. Organen und Oberflächen-Hüllgewebe (z.B. Organkapsel)
Variante 3) kommt in Betracht für Gewebestrukturen, die aus mehreren Zellarten zusammengesetzt sind. Beispielsweise bestehen Gefäße aus Endothel und Bindegewebe, wobei das Endothel mit flächiger Struktur zur Auskleidung eines Blutgefäßes dient, während das Bindegewebe als Stützsubstanz des Gefäßes fungiert und die dreidimensionale Hohlstruktur bildet. Durch die Kombination von 1) als Flächenelement für das Wachstum von Endothel und 2) als dreidimensionales Raumelement für das Wachstum von Bindegewebe kann letztendlich ein Gefäß rekonstruiert werden.

Erfindungsgemäß können für die einzelnen Anwendungen die folgenden Zellen/Gewebe ausgewählt werden.

Nachfolgend werden einige Gewebe- bzw. Zelltypen aufgelistet, die für die Vermehrung/Herstellung mittels einer der drei Varianten besonders geeignet sind. Für
- Anwendung 1 die folgenden Gewebe: Epithel, Endothel, Urothel, Mukosa, Dura, Bindegewebe; und die folgenden Zellen: pluripotente Stammzellen, Chondrozyten (Knorpel; zur Chondrozyten-Vermehrung braucht man ein zweidimensionales Medium, zur Chondrozyten-Differenzierung und Knorpelmatrix-Bildung hingegen braucht man ein dreidimensionales Medium. Hier sind bezüglich Knorpel nur die Zellen gemeint, wenn sie dedifferenzieren und sich vermehren. Die Differenzierung folgt in Anwendung 2), Osteozyten (Knochen; entweder zwei- oder dreidimensional, hier gilt dasselbe wie für die Chondrozyten), Nervenzellen (Nerven), Haarzellen (Innenohr-Hörorgan) oder deren Vorläuferzellen jeglicher Differenzierungsstufe (z.B. pluripotente Stammzellen).
- Anwendung 2 die folgenden Zellen: die für Anwendung 1) beschriebenen Zellen nach ihrer flächigen Vermehrung, organspezifische Zellen (z.B. Hepatozyten, Nephrozyten, Kardiomyozyten, Pankreozyten), Zellen des ZNS mit/ohne endokrine/r Funktion, z.B. Netzhaut, Neurozyten, Zirbeldrüse, dopaminerge Zellen, Gefäß bildende Zellen (z.B. Angiozyten), Zellen mit endo- oder exokriner Funktion (z.B. Inselzellen, Nebennierenzellen, Speicheldrüsenzellen, Epithelkörperchen, Thyrozyten), Zellen des Immunsystems (z.B. Makrophagen, B-Zellen, T-Zellen oder deren Vorläuferzellen jeglicher Differenzierungsstufe wie pluripotente Stammzellen). Die Zellen des Immunsystems werden dreidimensional gezüchtet, weil sie im Gewebe, nach Penetration der Blut-Gewebeschranke auf ein dreidimensionales Gerüst (je nach Gewebeart) treffen und dort im Dreidimensionalen ihre Wirkung entfalten.
- Anwendung 3 die folgenden Zellen/Gewebe/Organe: Trachea, Bronchien, Gefäße, Lymphgewebe, Harnröhre, Harnleiter, Niere, Blase, Nebenniere, Leber, Milz, Herz, Gefäße,
   Schilddrüse, Tonsillen, Speicheldrüsen, Gehirn, Muskel (glatt, quergestreift), Bandscheiben,
   Meniskus, Herz, Lunge, Galle, Speiseröhre, Darm, Auge.

In keiner der drei Anwendungen werden Zellen/Gewebe zur Hautherstellung verwendet, obwohl teilweise Fibroblasten verwendet werden. Aus diesen ließe sich zwar Haut herstellen, erfindungsgemäß dienen die Fibroblasten aber nur als bindegewebiges Stützgerüst, z.B. für Gefäße oder Organe.

### Beispiel 1

### Herstellung einer zweidimensionalen Chondrozytenkultur aus einer Knorpelbiopsie: Zellisolierung und Kultivierung

Nasenseptumknorpel wurde von Perichondrium befreit und in 1 mm³ Stücke zerteilt, in Hanks Lösung (Seromed, Berlin, Germany) gewaschen und mit 1 mg/ml Typ II Kollagenase (Seromed, Berlin, Germany), 0,1 mg/ml Hyaluronidase (Serva, Frankfurt, Germany) und 1,5 mg/ml Typ II DNase (Paesel, Frankfurt, Germany) 16 h in einer Kulturflasche mit Magnetrührer inkubiert. Die aus dem Knorpelmatrixverband gelösten Chondrozyten wurden nach der enzymatischen Behandlung von unverdauten Knorpelstücken mittels Filtration durch ein Nylonsieb (Porengröße von 200 µm; Reichelt Chemie, Heidelberg, Germany) getrennt. Nach Waschen der Zellsuspension in Hanks Lösung erfolgte die Zellmengenbestimmung mit einem Neubauer Hämatozytometer nach vorausgegangener Vitalitätsprüfung durch Trypan-Blau-Färbung.

### A) Monolayer-Chondrozytenkultur

Die Zellvermehrung erfolgt in 75 cm² Zellkulturflaschen (Nunc, Naperville, USA) mit modifi-ziertem RPMI 1640-Medium (500 ml RPMI mit 10% FCS, 10 ml HEPES, 5 ml Strep-tomycin/Penicillin), in die zuvor das erfindungsgemäße Trägermaterial eingelegt worden ist. Hierzu werden je Vermehrungsansatz beispielsweise 4-5 Mio Chondrozyten auf die Fasermatrix aufgebracht und in einem Brutschrank bei 37°C und 5,5% CO₂ inkubiert. Das Nährmedium wurde alle 2-3 Tage erneuert. Zur Passage werden die konfluenten Zellen mit einem 0,05%-igen Trypsin-EDTA Gemisch abgelöst. Hierunter kommt es zur Chondrozyten-Proliferation.

### B) Monolayer-Suspensionskultur humanen respiratorischen Epithels

Die zerkleinerten Mukosastücke werden nach Abspülen mit PBS (Gibco, Karlsruhe) im Kulturgefäß auf die Fasermatrix aufgebracht. Alternativ zusätzlich zum mechanischen Zerkleinerungsvorgang kann die Mukosa enzymatisch mit 0,1%iger Kollagenase-Lsg. (Seromed, Berlin) aufgespalten werden. Hierzu wird die Zellsuspension für ca. 4 Stunden bei 37°C inkubiert. Nach Spülen mit PBS und Zentrifugieren wird die Zellsuspension mit einer Zelldichte von ca. 5x10⁵ auf die Fasermatrix aufgebracht und mit modifiziertem Dulbecco's Eagle Medium unter Zusatz von Hepes-Puffer (1%), Penicillin-Streptomycin (1%) (alle Gibco, Karlsruhe) und fetalen Kälberserum (Seromed, Berlin) inkubiert.

### Beispiel 2

### Herstellung einer dreidimensionalen Chondrozytenkultur (Knorpelgewebedifferenzierung) aus einer Knorpelbiopsie

Die Isolierung und Vermehrung der Chondrozyten erfolgt entsprechend Beispiel 1. Für die 3-dimensionale Chondrozytenkultur werden einerseits in Monolayerkultur über 3 Passagen vermehrte Chondrozyten, andererseits native Chondrozyten in einer Konzentration von 1x10⁶ Zellen/ml in die Fasermatrix eingebracht und mit einem modifizierten RPMI 1640 Medium (500 ml RPMI mit 10% FCS, 10 ml HEPES, 5 ml Streptomycin/Penicillin) perfundiert. Hierunter kommt es zur Gewebediffenzierung und Knorpelbildung.

### Beispiel 3

### Herstellung eines Chondrozyten-/Schleimhaut-Verbundes i.S. eines Organes (Composite graft) aus einer Chondrozytenbiopsie und einer Schleimhautbiopsie (Beispiel für Variante 3)

Die Vermehrung bzw. die folgende dreidimensionale Chondrozytenanordnung erfolgt wie in Beispiel zu 1 A) und 2) beschrieben. Parallel dazu wird aus einer Schleimhautbiopsie (z.B. Nasenmuschel) Mukosa entsprechend Beispiel 1 B) aufgearbeitet und zweidimensional auf die dreidimensionale Oberfläche der Fasermatrix-Chondrozytenkombination aufgebracht. Die Kulturbedingungen sind analog zu den in 1A), 1B) und 2) beschriebenen Kulturbedingungen.

### Weiter Anwendungen

Eine weitere Anwendungsmöglichkeit des in der Erfindung zur Anwendung kommenden Materials ist die Besiedlung des Materials mit Zellen, die eine endo- bzw. exokrine Funktion besitzen und Wirkstoffe (z.B. Hormone, Interleukine, Entzündungsmediatoren, Enzyme) freisetzen, die eine Wirkung im Organismus oder außerhalb des entfalten. Das heißt, das erfindungsgemäß verwendete Material kann, bei seiner Besiedelung mit Zellen mit endo- oder exokriner Funktion auch außerhalb des Körpers zur Herstellung der o.g. Wirkstoffe dienen, die dann über bekannte Verfahren als Arzneimittel dem Körper zur Verfügung gestellt werden. Eine außerhalb des Körpers entfaltete Wirkung kann dazu dienen, Gewebe oder Zellen mit der freigesetzten Substanz zu beeinflussen. Wiederum eine weitere Anwendung der erfindungsgemäß verwendeten Matrix (Faser-Material) besteht in ihrer Verwendung als Wundschutz- und Wundverschluß im Sinne eines Pflasters oder Verbandes. Hierzu wird das Faser-Material als Flächenelement direkt oder zusammen mit die Wundheilung fördernden Substanzen und Medikamenten auf die Wunde (außen) aufgelegt.

Eine weitere Verwendung der Matrix ist die als bioresorbierbares Bio-Implantat als Leitschiene für die körpereigene Wundheilung unter oder auf Niveau der Haut/Schleimhaut bzw. im Körperinneren im Rahmen von Operationen an Organen und Geweben. Hierzu wird das Material falls möglich durch einen Arzt als Flächenelement oder dreidimensionales Raumelement direkt oder zusammen mit die Wundheilung fördernden Substanzen oder Medikamenten in die Wunde oder Organe/Gewebe, beispielsweise während einer Operation, eingebracht.

Die Eigenschaften des erfindungsgemäß verwendeten bioresorbierbaren, anorganischen Materials in Form von Fasern bedingen für die zu züchtenden Zellen eine nur geringe Änderung des Gewebemilieus, insbesondere entsteht kein saures Milieu, mit der Folge, dass eine negative Beeinflussung der Gewebe- und Organdifferenzierung verhindert wird. Weiterhin kommt es, unabhängig vom pH-Wert des Gewebes, zu einem vollständigen Abbau des Materials. Durch den gleichzeitig stattfindenden Gewebe- bzw. Organaufbau findet sich immerzu vitales Gewebe mit der Möglichkeit der Penetration durch antiinfektiöse Medikamente bei ungewollter Besiedlung mit Erregern (Infektion).

Darüber hinaus kann die Fasermatrix mit Wirkstoffen unterschiedlicher Substanzgruppen versetzt werden mit der Möglichkeit einer positiven Beeinflussung der Gewebe- und Organdifferenzierung durch Entfaltung einer aktiven und passiven Wirkung am Ort der Anwendung, aber auch durch Wirkungsentfaltung an einem entfernt liegenden Wirkort. Hierzu zählen insbesondere einerseits antiinfektiöse Wirkstoffe, andererseits aber auch die Wundheilung, die Entzündungsreaktion sowie die Gewebedifferenzierung unterstützende und modulierende Wirkstoffe wie beispielsweise einerseits Wachstumsfaktoren (IGF, TGF, FGF etc.), andererseits Glukocortikoide und Interleukine, aber auch Chemotherapeutika und Immunsuppressiva.

Die erfindungsgemäß verwendeten bioresorbierbaren, anorganischen Fasern ermöglichen eine Anhaftung der zur Anwendung kommenden Zellen mit der Möglichkeit zur Vermehrung der Zellen entlang der Fasern, aber auch mit der Möglichkeit der Ausbildung einer Gewebe- bzw. Organmatrix. Gleichzeitig mit der Vermehrung der Zellen bzw. der Ausbildung einer Gewebe- bzw. Organmatrix kommt es zum Abbau der Faserstruktur. Idealerweise wird der Gewebe-, Organ- bzw. Zellaufbau mit der Abbaurate des Fasermaterials durch Variation der Kondensation der Fasern korreliert. Dabei gilt, je geringer der Kondensationsprozess (d.h., die Abspaltung von Wasser und damit die Polykondensation) fortgeschritten ist, umso besser kann das Material abgebaut werden. Der höchste OH-Gehalt und damit die am schnellsten abbaubare Faser erhält man bei frisch gesponnenen Fasern, die anschließend in Ethanol eingelegt sind. Der Kondensationsprozeß wird außerdem von den Spinnparametern beeinflusst, d.h. Abziehrate, Atmosphäre, Spinntemperatur etc. So hergestellte Fasern sind biologisch degradierbar und biologisch resorbierbar und lösen sich in schwach basischen, körperähnlichen Flüssigkeiten mit Abbauraten von 10 nm bis 100 nm Faserradius pro Tag auf, wobei die Abbaurate mit der Zahl der Silanol-Gruppen der Faser korreliert ist.

Ein weiterer Aspekt der vorliegenden Erfindung betrifft die Verwendung der erfindungsgemäßen Zellen, Organe und Gewebe, nachdem sie mit Medikamenten und/oder Wirkstoffen versetzt worden sind, als *in vitro*-Modell für Medikament-Gewebe-Organinteraktionen. Hierdurch können tierexperimentelle Untersuchungen minimiert bzw. vermieden werden.

Eine erste Ausführungsform der Erfindung gemäß Stammanmeldung betrifft ein Verfahren zur in vitro-Herstellung von Zellen, Geweben und Organen, wobei die in der Patentschrift DE 196 09 551 C1 erwähnte Fasermatrix als Zellstützsubstanz und/oder Leitstruktur für die von den Zellen gebildete extrazelluläre Matrix dient bzw. den Zellen die Möglichkeit gibt, eine räumliche Anordnung zu finden, die es den Zellen erlaubt, sich zu vermehren und/oder ihre genetisch determinierte Differenzierung zu erreichen.

Die Erfindung betrifft in einer bevorzugten Ausführungsform gemäß Stammanmeldung ein Verfahren zur in vitro-Herstellung von Zellen, Geweben und Organen, wobei die auf die Matrix aufzubringenden Zellen sich vornehmlich zweidimensional auf der Fasermatrix als Leitstruktur vermehren oder in der Matrix anhaften, um eine extrazelluläre Matrix bzw. Botenstoffe (Hormone) zu bilden.

In einer bevorzugten Ausführungsform der Erfindung gemäß Stammanmeldung ist die Matrix porös ist, so dass die ein-/aufgebrachten Zellen sie penetrieren, eine dreidimensionale Verteilung annehmen und entsprechend ihrer genetisch determinierten oder durch hinzu gegebene Differenzierungsfaktoren induzierten Differenzierung ein räumliches Gewebe- und Organwachstum auslösen können bzw. Botenstoffe freisetzen.

In einer weiteren bevorzugten Ausführungsform der Erfindung gemäß Stammanmeldung ist die Matrix als dichtes, von den ein-/aufgebrachten Zellen nicht penetrierbares Fasernetz mit der Möglichkeit der zweidimensionalen Zellverteilung und der gleichzeitigen Möglichkeit eines dreidimensionalen Gewebe- und Organwachstums im Sinne eines Composite grafts ausgebildet.

Weiter bevorzugt gemäß Stammanmeldung sind Zellen, Gewebe oder Organ, herstellbar mittels des erfindungsgemäßen Verfahrens.

Vorzugsweise werden gemäß Stammanmeldung die erfindungsgemäßen Zellen, Gewebe oder Organ(e) als in vitro-Modell für Medikament-Gewebe-Organinteraktionen verwendet.

Eine weitere Ausführungsform der Erfindung gemäß Stammanmeldung verwendet die oben beschriebene Fasermatrix als Wundschutz-Pflaster oder Wundschutz-Verband.

Erfindungsgemäß bevorzugt wird diese Fasermatrix gemäß Stammanmeldung als Leitstruktur für das Zellwachstum oder für die Akkumulation extrazellulärer Matrix- und Gewebebestandteile verwendet.

## Patentansprüche

1. Flächenelement und/oder dreidimensionales Raumelement aus Zellen, hergestellt mittels Zellen und einer Fasermatrix aus biologisch degradierbaren und/oder biologisch resorbierbaren Fasern, wobei die Fasermatrix als Zellstützsubstanz und/oder Leitstruktur für die von den Zellen gebildete extrazelluläre Matrix dient bzw. den Zellen die Möglichkeit gibt, eine räumliche Anordnung zu finden, die es den Zellen erlaubt, sich zu vermehren und/oder ihre genetisch determinierte Differenzierung zu erreichen, und die biologisch degradierbaren und/oder biologisch resorbierbaren Fasern **dadurch** erhältlich sind, dass man aus einer Spinnmasse Fäden zieht und diese gegebenenfalls trocknet, wobei die Spinnmasse eine oder mehrere partiell hydrolytisch kondensierte Verbindungen des Siliziums enthält, die sich durch hydrolytische Kondensation ableiten von Monomeren der allgemeinen Formel SiX₄, in der die Reste X gleich oder verschieden sind und Hydroxy, Wasserstoff, Halogen, Amino, Alkoxy, Acyloxy, Alkylcarbonyl oder Alkoxycarbonyl bedeuten und sich von Alkyl-Resten ableiten, die gegebenenfalls substituierte geradkettige, verzweigte oder cyclische Reste mit 1 bis 20 Kohlenstoff-Atomen, bevorzugt mit 1 bis 10 Kohlenstoff-Atomen, darstellen und durch Sauerstoff- oder Schwefel-Atome oder durch Amino-Gruppen unterbrochen sein können.
